(19) Europäisches Patentamt
European Patent Office
Office européen des brevets

(11) EP 1 048 737 B1

(12) EUROPÄISCHE PATENTSCHRIFT

(45) Veröffentlichungstag und Bekanntmachung des Hinweises auf die Patenterteilung:
**06.06.2007 Patentblatt 2007/23**

(51) Int Cl.:
***C12P 7/26*** *(2006.01)* ***C12P 13/00*** *(2006.01)*
***C12P 17/00*** *(2006.01)* ***C12P 17/12*** *(2006.01)*
***C12P 17/04*** *(2006.01)* *C12N 9/88* *(2006.01)*

(21) Anmeldenummer: **00108709.7**

(22) Anmeldetag: **22.04.2000**

(54) **Stereoselektive Synthese von 2-Hydroxyketonen**

Stereoselective synthesis of 2-hydroxycétones

Procédé pour la synthèse stéréosélective de 2-hydroxyketones

(84) Benannte Vertragsstaaten:
**CH DE FR GB LI**

(30) Priorität: **27.04.1999 DE 19918935**

(43) Veröffentlichungstag der Anmeldung:
**02.11.2000 Patentblatt 2000/44**

(73) Patentinhaber: **Forschungszentrum Jülich GmbH 52425 Jülich (DE)**

(72) Erfinder:
- **Dünnwald, Thomas 53115 Bonn (DE)**
- **Greiner, Lasse 52351 Düren (DE)**
- **Iding, Hans, Dr. 79618 Rheinfelden (DE)**
- **Liese, Andreas, Dr. 52428 Jülich (DE)**
- **Müller, Michael, Dr. 52428 Jülich (DE)**
- **Pohl, Martina, Dr. 52066 Aachen (DE)**

(56) Entgegenhaltungen:
**DE-A- 19 523 269** **DE-A- 19 736 104**

- **SPRENGER GEORG A ET AL: "Synthetic potential of thiamin diphosphate-dependent enzymes." JOURNAL OF MOLECULAR CATALYSIS B ENZYMATIC, Bd. 6, Nr. 3, 11. März 1999 (1999-03-11), Seiten 145-159, XP000938953 ISSN: 1381-1177**
- **WILCOCKS R ET AL: "ACYLOIN FORMATION BY BENZOYLFORMATE DECARBOXYLASE FROM PSEUDOMONAS-PUTIDA" APPLIED AND ENVIRONMENTAL MICROBIOLOGY, Bd. 58, Nr. 5, 1992, Seiten 1699-1704, XP000938916 ISSN: 0099-2240**
- **WILCOCKS R ET AL: "FACTORS AFFECTING 2 HYDROXYPROPIOPHENONE FORMATION BY BENZOYLFORMATE DECARBOXYLASE FROM PSEUDOMONAS-PUTIDA" BIOTECHNOLOGY AND BIOENGINEERING, Bd. 39, Nr. 10, 1992, Seiten 1058-1063, XP002147921 ISSN: 0006-3592**
- **IDING H ET AL: "Application of alpha-keto acid decarboxylases in biotransformations." BIOCHIMICA ET BIOPHYSICA ACTA, Bd. 1385, Nr. 2, 29. Juni 1998 (1998-06-29), Seiten 307-322, XP000938920 ISSN: 0006-3002**
- **S. BORNEMANN ET AL.: "Stereospecific formation of R-aromatic acyloins by Zymonas mobilis pyruvate decarboxylase" J. CHEM. SOC., PERKIN TRANS. I, Nr. 5, 7. März 1996 (1996-03-07), Seiten 425-430, XP002147922 London, GB**
- **A.S. DEMIR ET AL.: "Asymmetric benzoin reaction catalyzed by benzoylformate decarboxylase" TETRAHEDRON: ASMMETRY, Bd. 10, 17. Dezember 1999 (1999-12-17), Seiten 4769-4774, XP002147923 PERGAMON PRESS, ELSEVIER, NY, US**
- **H. IDING ET AL.: "Benzoylformate decarboxylase from Pseudomonas putida as stable catalyst for the synthesis of chiral 2-hydroxy ketones" CHEM. EUR. JOURNAL, Bd. 6, Nr. 8, 14. April 2000 (2000-04-14), Seiten 1483-1495, XP002147924 WILEY-VCH VERLAG, WEINHEIM, FRG**

EP 1 048 737 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- **T. DÜNNWALD ET AL.: "Enantioselective Synthesis of (S)-2-Hydroxypropanone derivatives by benzoylformate decarboxylase catalyzed C-C bond formation" EUR. J. ORG. CHEM., Nr. 11, Juni 2000 (2000-06), Seiten 2161-2170, XP002147925 WILEY-VCH VERLAG, WEINHEIM, FRG**

## Beschreibung

Die Erfindung betrifft ein Verfahren zur stereoselektiven Synthese von 2-Hydroxyketonen.

In R. Wilcocks, O.P. Ward, Biotech. Bioeng. 1992, 39, 1058-1063 und R. Wilcocks et al., Appl. Env. Microbiol., 1992, 58, 1699-1704 wird die Synthese von (S)-2-Hydroxy-1-phenyl-propanon (2-HPP) mit der Thiamindiphosphat (ThDP)-abhängigenBenzoylformiat-decarboxylase (BFD) mit Benzoylformiat und Acetaldehyd als Substrate in Gegenwart von ganzen Zellen und Zellextrakten aus *Pseudomonas putida* (*Ps. p.*) beschrieben. Allerdings entsteht dieses Produkt nicht enantiomerenrein, sondern lediglich mit einem Enantiomerenüberschuß von 92 %. Darüber hinaus wird Benzylalkohol als Nebenprodukt gebildet.

Ferner ist aus H. Iding, et al., Biochim. Biophys. Acta, 1998, 1385, 307-322 bekannt, daß auch Benzaldehyd selbst zur Carboligation mit Acetaldehyd eingesetzt werden kann.

In Iding et al., BBA, 1998, 1385, 307-322 und Sprenger und Pohl, J. Molec. Cat. B: Enzymatic, 1999, 6, 145-159 wurde das Potential von Thiamindiphosphat-abhängiger 2-Ketosäuredecarboxylasen für die Pyruvatdecarboxylase (PDC) genau untersucht. Die PDC katalysiert die Synthese von (*R*)-2-Hydroxyketonen.

Die Veröffentlichung "Synthetic potential of thiamin diphosphate-dependent enzymes" von Sprenger und Pohl im Journal of Molecular Catalysis B: Enzymatic 6 Nr. 3 vom 11. März 1999 (1999-03-11) Seite 145-159 offenbart, die Bildung von C-C-Bindungen bei 2-Hydroxyketonenmit Aldehyden und Ketonen als Anfangsverbindungen unter Verwendung von Benzoylformiatdecarboxylase aus *Pseudomonas putida.*

Die DE 197 36 104 A1 offenbart ein Verfahren zur Herstellung von enantiomerenreinen Phenylacetylcarbinolen in Gegenwart von Pyruvatdecarboxylase aus *Zymomonas.*

Die DE 195 23 269 A1 zeigt ein Verfahren zur Gewinnung von Acyloinen mittels Pyruvatdecarboxylase.

S. Bornemann et al. zeigt im J. Chem. Soc., Perkin Trans. I, Nr. 5, 7 (1996-03-07) Seite 425-430, dass rekombinante Pyruvatdecarboxylase aus *Zymomonas mobilis* die Bildung von aromatischen R-Acyloinen katalysiert.

Aufgabe der Erfindung ist es, ein einfaches Verfahren zur stereoselektiven Synthese von 2-Hydroxyketonen zu schaffen, welches auf ein weites Subtratspektrum angewandt werden kann, und bei dem eine Steigerung der Enantioselektivität durch Variation der Verfahrensparameter möglich ist.

Die Aufgabe wird gelöst durch ein Verfahren gemäß Anspruch 1. Vorteilhafte Ausgestaltungen des Verfahrens ergeben sich aus den rückbezogenen Ansprüchen.

Das anspruchsgemäße Verfahren zur Synthese von 2-Hydroxyketonen setzt als ein erstes Substrat ein Aldehyd, ausgenommen Benzaldehyd, oder eine 2-Ketosäure, ausgenommen Benzoylformiat, ein.
Als ein zweites Substrat wird ebenfalls ein Aldehyd, ausgenommen Benzaldehyd, eingesetzt. Geeignete Aldehyde sind beispielsweise substituierte Benzaldehyde, heteroaromatische Aldehyde, aber auch nicht-aromatische Aldehyde, wie z.B. cyclische Aldehyde und auch lineare Aldehyde. Geeignete 2-Ketosäuren sind u.a. 2-Oxopentansäure, 3-Methoxyphenylglyoxylsäure, 4-Methylphenylglyoxylsäure und Fur-2-ylglyoxylsäure

Die enzymatische Reaktion der Substrate zu 2-Hydroxyketonen erfolgt in Gegenwart von Thiamindiphosphat (ThDP) - abhängigen Enzymen, welche eine C-C-Verknüpfung zwischen den zwei Substraten bewirken. Zu den Thiamindiphosphat(ThDP)-abhängigen Enzymen gehören beispielsweise die Benzoylformiatdecarboxylase (BFD) und die Pyruvatdecarboxylase (PDC).

Im Fall einer 2-Ketosäure als ein erstes Substrat, wird diese zunächst decarboxyliert und es entsteht ein Thiamindiphosphat-gebundener Acyldonor (Aldehyd). Dieser wird anschließend in Gegenwart eines Enzyms auf das als Acylakzeptor fungierende zweite Substrat so übertragen, daß ein 2-Hydroxyketon entsteht. In dem Fall, daß als ein erstes Substrat schon ein Aldehyd eingesetzt wird, geschieht die Synthese mit einem zweiten Substrat in Gegenwart eines Enzyms direkt zu einem 2-Hydroxyketon

Die Bildung von 2-Hydroxyketonen erfolgt in diesem anspruchsgemäßen Verfahren stereoselektiv, bzw. enantioselektiv. Das heißt, daß von den zwei möglichen Stereoisomeren (Enantiomeren) des gebildeten 2-Hydroxyketons, in diesem Fall (S) und (R)-Enantiomere, ein Isomer regelmäßig überwiegt. Ein Maß für die Selektivität ist der sogenannte Enantiomerenüberschuß (ee). Darunter ist der Betrag der Differenz der prozentualen Ausbeuten der beiden vorkommenden Isomere (S und R) im Produkt zu verstehen. In diesem Fall ergibt sich der Enantiomerenüberschuß ee = Betrag (%S - %R) = (S-R)/(S+R). Liegt ein Isomer zu 20 % vor und das andere Isomer zu 80 %, so ergibt sich der Enantiomerenüberschuß zu 60 %. Des weiteren zeigen sich bei dem anspruchsgemäßen Verfahren keinerlei chemische Nebenreaktionen.

In einer vorteilhaften Ausgestaltung wird das Verfahren in Gegenwart einer 2-Ketosäuredecarboxylase durchgeführt.

Vorteilhaft wird das Verfahren in Gegenwart einer Benzoylformiatdecarboxylase durchgeführt.

Ebenfalls von Vorteil ist der Einsatz einer Benzoylformiatdecarboxylase aus *Pseudomonas putida*.

Vorteilhaft wird als ein zweites Substrat ein Acetaldehyd eingesetzt.

Für das anspruchsgemäße Verfahren lassen sich eine große Klasse von Substanzen als ein erstes Substrat einsetzen. Zu diesen gehören beispielsweise unsubstituierte aromatische Aldehyde, substituierte aromatische Aldehyde,

heteroaromatische und substituierte, heteroaromatische Aldehyde, aber auch cyclische und nichtcyclische, aliphatische oder olefinische Aldehyde. Dieses weite Einsatzspektrum eröffnet die Möglichkeit, entsprechend vielfältige 2-Hydroxyketone in (S) Konfiguration mit hoher Selektivität durch das anspruchsgemäße Verfahren einfach zu synthetisieren.

Es wurde gefunden, daß bei dem anspruchsgemäßen Verfahren eine Steigerung der Enantioselektivität durch Wahl geeigneter Substrate, z.B. bei in 3- oder 4-Position substituierten Benzaldehyden, erzielt werden kann. Als Substituenten sind beispielsweise -OBn, -OAc, -OPh oder auch -O*i*Pr geeignet. Bei Ihnen liegt die Enantioselektivität regelmäßig > 99%. Auch Substituenten wie -Me - OMe oder -OEt führen zu einer höheren Selektivität verglichen mit der eines unsubstituierten Benzaldehyds. In einer vorteilhaften Verfahrensausgestaltung können daher in 3- oder 4-Position substituierte Benzaldehyde als ein erstes Substrat eingesetzt werden.

Eine weitere vorteilhafte Ausgestaltung des Verfahrens setzt nicht-aromatische Aldehyde als ein erstes Substrat ein. Mit dieser Substratklasse war bislang noch keine enzymatische Kreuzkupplung, wie die erfindungsgemäße Synthese von 2-Hydroxyketonen, gelungen.

Die Enantioselektivität läßt sich bei dem anspruchsgemäßen Verfahren durch geeignete Wahl der Konzentration der eingesetzten Substrate sowie einer geeigneten Temperatur während des Verfahrens regelmäßig steigern.
Es stellte sich überraschend heraus, daß die Steigerung der Enantioselektivität bei der anspruchsgemäßen enzymatischen Bildung von 2-Hydroxy-1-phenyl-propanon (2-HPP) durch eine Verringerung der Konzentration an Benzaldehyd bzw. durch eine Erhöhung der Konzentration an Acetaldehyd erreicht werden kann.
Die Konzentration des ersten Substrates liegt vorteilhaft unter 500 mM, insbesondere unter 50 mM. Die Enantioselektivität wird regelmäßig um so größer, je kleiner die Konzentration des zweiten Substrates ist. Daher ist es vorteilhaft, möglichst kleine Konzentrationen in dem Verfahren zu realisieren. Die Untergrenze für die Konzentration des zweiten Substrates ist verfahrensabhängig und liegt aus praktischen Gründen bei ca. 1 μM. Eine obere Konzentration des zweiten Substrates von ca. 1 M bewirkt regelmäßig eine Deaktivierung des Enzyms. Eine vorteilhafte Verfahrensausgestaltung sieht daher Konzentrationen für das zweite Substrat vor, die kleiner als 1 M, insbesondere kleiner als 500 mM sind. Der typische Arbeitsbereich liegt aus verfahrenstechnischen Gründen bei 100-500 mM, unterschreitet jedoch regelmäßig 1 mM nicht.

Diese oben genannten vorteilhaften Konzentrationsbereiche für das erste und zweite Substrat ermöglichen einen hohen Enantiomerenüberschuß.

Ein typischer Temperaturbereich für enzymatische Verfahren liegt im Bereich von -10 ˚C bis 60 ˚C. Es hat sich herausgestellt, daß die Enantioselektivität bei dem anspruchsgemäßen Verfahren eine Temperaturabhängigkeit aufweist. Dabei steigt die Selektivität regelmäßig mit sinkender Temperatur. In einer vorteilhaften Ausgestaltung des Verfahrens wird daher während des Verfahrens eine Temperatur unterhalb von 30 ˚C, insbesondere unterhalb von 25 ˚C eingestellt. Die untere Temperaturgrenze wird durch das System festgelegt. Sie liegt typischerweise bei -10 ˚C und ist substratabhängig.

Die Erfindung wird im Folgenden anhand von einer Tabelle, von Figuren sowie Ausführungsbeispielen näher erläutert.

Tabelle 1: Substrat- und Produktspektrum der BFD aus *Ps. p.* für die Carboligation. Die absolute Konfiguration der 2-Hydroxyketone ist (S).

Figur 1: chirale 2-Hydroxyketone als Zwischenprodukte für die organische Synthese.

Figur 2: Enantiomerenüberschuß als Funktion der Benzaldehydkonzentration und der Temperatur (500 mM Acetaldehyd, 0,5 mM ThDP, 0,05 mM MgCl2, 0,17 U/ml BFD, 50 mM Phosphatpuffer ph7)

Figur 3: Enantiomerenüberschuß als Funktion der Acetaldehydkonzentration (0,5 mM ThDP, 0,05 mM MgCl2, 0,17 U/ml BFD, 50 mM Phosphatpuffer ph7)

Figur 4: Enantiomerenüberschuß als Funktion des Umsatzes im Stahl-EMR (40 mM Benzaldehyd, 500 mM Acetaldehyd, 0,5 mM ThDP, 1 mM $MgCl_2$, 6,5 U/ml BFD, 50 mM Phosphatpuffer pH7, 25 ˚C)

Figur 5: Umsatz und ee als Funktion der Zeit im Polypropylen-EMR (10 mM Benzaldehyd, 100 mM Acetaldehyd, 4 mM ThDP, 0,5 mM $MgCl_2$, 5 U/ml BFD, 50 mM Phosphatpuffer pH7)

Figur 6: ee als Funktion der Zeit im Polypropylen-EMR für zwei verschiedene Betriebstemperaturen unter sonst gleichen Bedingungen (10 mM Benzaldehyd, 100 mM Acetaldehyd, 4 mM ThDP, 0,5 mM $MgCl_2$, 5 U/ml BFD, 50 mM Phosphatpuffer pH7)

Die vorliegende Erfindung betrifft die enantioselektive Synthese chiraler 2-Hydroxyketone mittels Benzoylformiatdecarboxylase. Chirale 2-Hydroxyketone sind wichtige Zwischenprodukte in der enantioselektiven organischen Synthese. Das synthetische Potential ist in Fig. 1 gezeigt.

Das Potential Thiamindiphosphat-abhängiger 2-Ketosäuredecarboxylasen ist bisher nur für die Pyruvatdecarboxylase (PDC) genau untersucht (Iding et al., BBA, 1998, 1385, 307-322; Sprenger und Pohl, J. Molec. Cat. B: Enzymatic, 1999, 6, 145-159). Die PDC katalysiert die Synthese von (*R*)-2-Hydroxyketonen des Typs der allgemeinen Formel I.

I

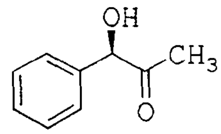

Alle bisher charakterisierten mittels PDC gewonnenen 2-Hydroxyketone dieses Typs entstehen enantiomerenrein.

Wilcocks et al. (App. Env. Microbiol., 1992, 58, 1699-1704) und Wilcocks und Ward (Biotech. Bioeng., 1992, 39, 1058-1063) beschreiben (*S*)-2-Hydroxypropiophenon (Formel II) als Reaktionsprodukt der Decarboxylierung von Benzoylformiat in Gegenwart von Acetaldehyd in Gegenwart ganzer Zellen von *Pseudomonas putida* (BFD aus *Ps. p.*) bzw. von Rohextrakten dieser Zellen. Sie führen die 2-Hydroxypropiophenonsynthese auf das Enzym Benzoylformiatdecarboxylase zurück.

II

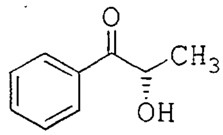

Allerdings entsteht dieses Produkt nicht enantiomerenrein, sondern lediglich mit einem Enantiomerenüberschuß von 92%. Darüber hinaus wird Benzylalkohol als Nebenprodukt gebildet, bedingt durch die Anwesenheit einer entsprechenden Benzaldehyddehydrogenase in den *Ps. putida*-Zellen, wie auch im Rohextrakt.

Wir haben festgestellt, daß die Erzeugung des Nebenprodukts Benzylalkohol durch eine einfache chromatographische Reinigung des Rohextrakts vermeidbar ist. Außerdem gelingt auf diesem Wege eine wirkungsvolle Anreicherung der BFD. Allerdings konnte durch die Reinigung der Enzymfaktion keine Verbesserung der optischen Reinheit des (*S*)-2-Hydroxypropiophenons erzielt werden.

Andere durch BFD-Katalyse erzielten 2-Hydroxyketone wurden bisher nicht beschrieben.
Wir haben nun überraschend festgestellt, daß das synthetische Potential der BFD durch die Verwendung von Aldehyden als Acyldonor wesentlich größer ist als bisher bekannt. Unsere Arbeiten zeigen, daß die primäre Decarboxylierung einer 2-Ketosäure zur Erzeugung eines Thiamindiphosphat-gebundenen Acyldonors nicht notwendig sind. Vielmehr wird diese Spezies auch durch Zugabe des korrespondierenden Aldehyds zur BFD gebildet. Die Reaktionsprodukte aus der erfindungsgemäßen Verknüpfung verschiedener Acyldonor-Aldehyde mit Acetaldehyd als Acylakzeptor entsprechen der allgemeinen Formel III. Alle Reaktionsprodukte des Typs III zeigen (S)-Konfiguration am chiralen Zentrum.

III

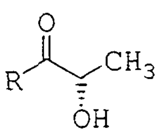

Die in Satzreaktoren erhaltenen Produkte und Enantiomerenüberschüsse gibt Tab. 1 wieder.

Hieraus ist ersichtlich, daß aus der bereits publizierten BFD-katalysierten Darstellung von (R)-Benzoin, ausgehend von zwei Molekülen Benzaldehyd, weder auf die von uns gefundene breite Substratvarianz des Donoraldehyds (dieses bleibt im Falle der (R)-Benzoin-Darstellung Benzaldehyd) noch auf die Abhängigkeit des stereochemischen Verlaufs der enzymatischen Reaktion von Temperatur, Konzentration und Substitutionsmuster geschlossen werden kann.

Tabelle 1: Substrat- und Produktspektrum der BFD aus *Ps. putida* für die Carboligation. Die absolute Konfiguration der 2-Hydroxyketone ist (S).

| | **Acyldonor** | **Produkt** | **Umsatz nach 20 h (%)** | **ee (%) HPLC [min]** |
|---|---|---|---|---|
| Unsubstituierte aromatische Aldehyde | | | | |
| 1. | 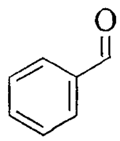 | 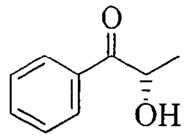<br>GCMS: $R_t$ = 7.68 min<br>m/z = 150 ($M^+$) | 100 | 92<br>$R_t$ (*S*) = 12.79<br>$R_t$ (*R*) = 20.23 |
| 2. | 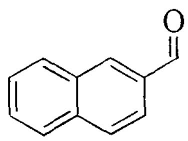 | 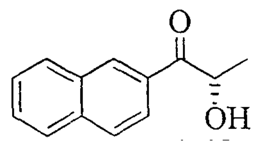<br>GCMS: $R_t$ = 11.25 min<br>m/z = 200 ($M^+$) | 98 | 89<br>$R_t$ (*S*) = 14.58<br>$R_t$ (*R*) = 17.43 |
| Substituierte aromatische Aldehyde | | | | |
| 3. | 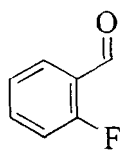 | 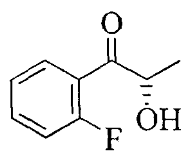<br>GCMS: $R_t$ = 7.46 min<br>m/z = 168 ($M^+$) | 100 | 89<br>$R_t$ (*S*) = 10.82<br>$R_t$ (*R*) = 15.24 |
| 4. | 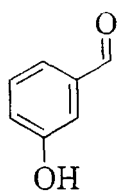 | 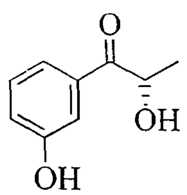<br>GCMS: $R_t$ = 9.99 min<br>m/z = 166 ($M^+$) | 83 | 88<br>$R_t$ (*S*) = 12.90<br>$R_t$ (*R*) = 14.36 |
| 5. | 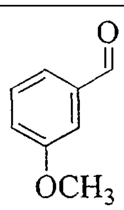 | 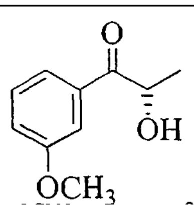<br>GCMS: $R_t$ = 9.33 min<br>m/z = 180 ($M^+$) | 100 | 96<br>$R_t$ (*S*) = 17.75<br>$R_t$ (*R*) = 32.67 |
| 6. | 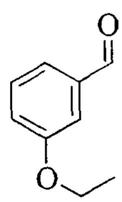 | 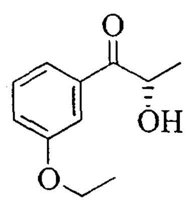<br>GCMS: $R_t$ = 9.73 min<br>m/z = 194 ($M^+$) | 100 | 97<br>$R_t$ (*S*) = 14.58<br>$R_t$ (*R*) = 34.96 |

(fortgesetzt)

| | Acyldonor | Produkt | Umsatz nach 20 h (%) | ee (%) HPLC [min] |
|---|---|---|---|---|
| 7. | 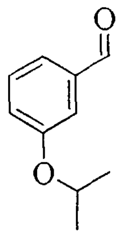 | 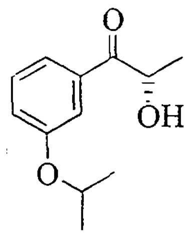 GCMS: $R_t$ = 9.87 min m/z = 208 ($M^+$) | 80 | > 99 $R_t$ (*S*) = 11.85 |
| 8. | 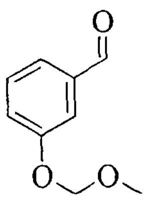 | 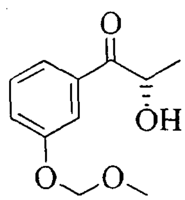 GCMS: $R_t$ =10.13 min m/z = 210 ($M^+$) | 88 | > 99 $R_t$ (*S*) = 25.73 |
| 9. | 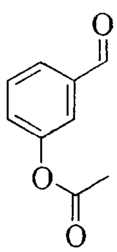 | 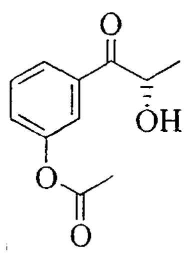 GCMS: $R_t$=10.10min m/z = 208 ($M^+$) | 48 | > 99 $R_t$ (*S*) =38.09 |
| 10. | 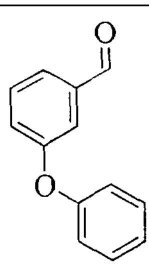 | 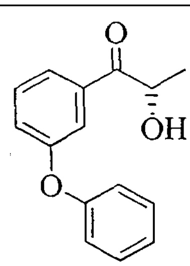 GCMS: $R_t$ = 12.05 min m/z = 242 ($M^+$) | 18 | > 99 $R_t$ (*S*) = 16.53 |
| 11. | 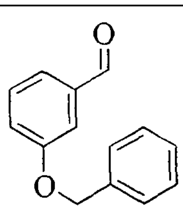 | 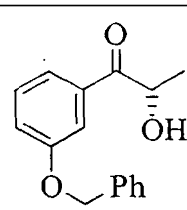 GCMS: $R_t$ = 12.89 min m/z =256 ($M^+$) | 97 | > 99 $R_t$ (*S*) = 34.06 |

(fortgesetzt)

| | Acyldonor | Produkt | Umsatz nach 20 h (%) | ee (%) HPLC [min] |
|---|---|---|---|---|
| 12. | 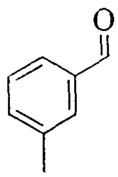 | 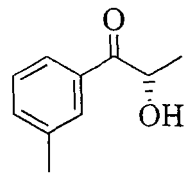<br>GCMS: $R_t$ =8.45 min<br>m/z = 164 ($M^+$) | 100 | 97<br>$R_t$ (*S*) = 9.93<br>$R_t$ (*R*) = 19.33 |
| 13. | 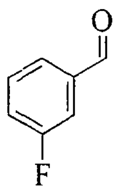 | 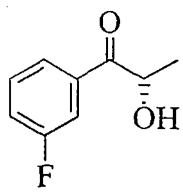<br>GCMS: $R_t$ = 7 . 52 min<br>m/z = 168 ($M^+$) | 100 | 86<br>$R_t$ (*S*) = 10.74<br>$R_t$ (*R*) =14.60 |
| 14. | 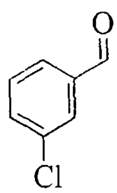 | 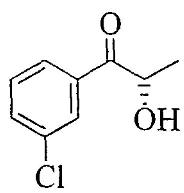<br>GCMS: $R_t$ = 8.87 min<br>m/z = 186 ($M^+$), 184 ($M^+$) | 100 | 94<br>$R_t$ (*S*) = 10.96<br>$R_t$ (*R*) = 17.15 |
| 15. | 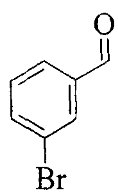 | 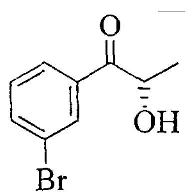<br>GCMS: $R_t$ = 9.49 min<br>m/z = 229 ($M^+$), 227 ($M^+$) | 100 | 96<br>$R_t$ (*S*) = 12.46<br>$R_t$ (*R*) = 19.00 |
| 16. | 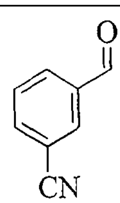 | 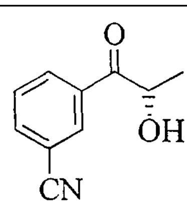<br>GCMS : $R_t$ = 9.76 min<br>m/z = 175 ($M^+$) | 68 | 92<br>$R_t$ (*S*) = 39.79<br>$R_t$ (*R*) = 56.59 |
| 17. | 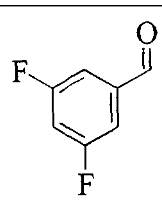 | 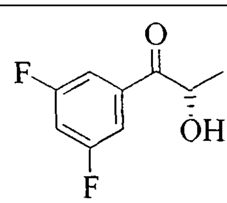<br>GCMS: $R_t$ = 7.06 min<br>m/z = 186 ($M^+$) | 100 | 80<br>$R_t$ (*S*) = 8.68<br>$R_t$ (*R*) = 10.99 |

(fortgesetzt)

| | Acyldonor | Produkt | Umsatz nach 20 h (%) | ee (%) HPLC [min] |
|---|---|---|---|---|
| 18. | 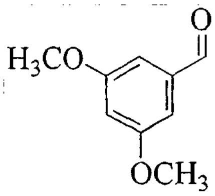 | 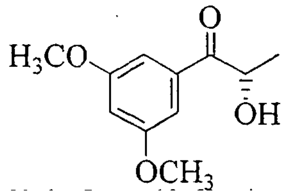 GCMS: $R_t$ = 10.61 min m/z = 210 ($M^+$) | 44 | > 99 $R_t$ (*S*) = 21.10 |
| 19. | 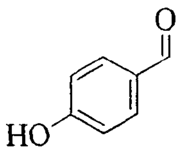 | 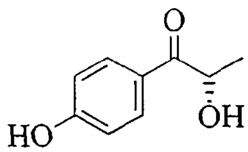 GCMS: $R_t$ =10.34 min m/z = 166 ($M^+$) | 12 | 95 $R_t$ (*S*) =15.56 $R_t$ (*R*) = 21.17 |
| 20. | 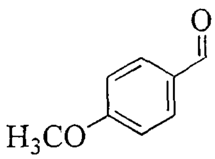 | 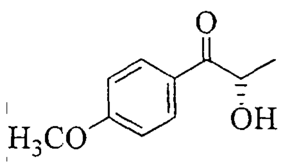 GCMS: $R_t$ = 9.71 min m/z =180 ($M^+$) | 37 | >99 $R_t$ (*S*) = 20.16 $R_t$ (*R*) = 30.22 |
| 21. | 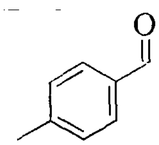 | 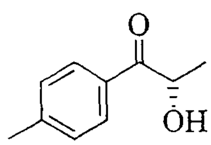 GCMS: $R_t$ = 8.58 min m/z =164 ($M^+$) | 83 | 88 $R_t$ (*S*) = 10.83 $R_t$ (*R*) = 19.55 |
| 22. | 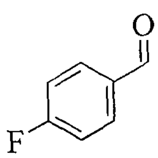 | 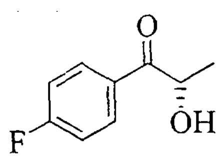 GCMS: $R_t$ = 7.60 min m/z =168 ($M^+$) | 76 | 87 $R_t$ (*S*) = 11.56 $R_t$ (*R*) =18.10 |
| 23. | 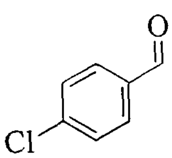 | 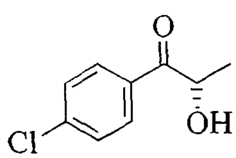 GCMS: $R_t$ =8.93 min m/z = 186 ($M^+$), 184 ($M^+$) | 49 | 82 $R_t$ (*S*) = 10.97 $R_t$ (*R*) =14.74 |

(fortgesetzt)

| | Acyldonor | Produkt | Umsatz nach 20 h (%) | ee (%) HPLC [min] |
|---|---|---|---|---|
| 24. | 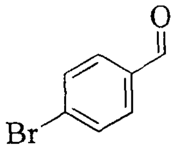 | 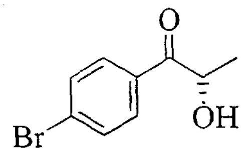 GCMS: $R_t$ = 9.57 min m/z = 229 ($M^+$), 227 ($M^+$) | 96 | 83 $R_t$ (*S*) = 11.45 $R_t$ (*R*) =14.38 |
| 25. | 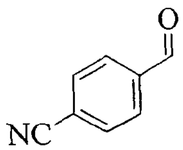 | 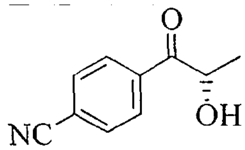 GCMS: $R_t$ =9.73 min m/z = 175 ($M^+$) | 44 | 74 $R_t$ (*S*) =37.87 $R_t$ (*R*) = 52.35 |
| Heteroaromatische Aldehyde | | | | |
| 26. | 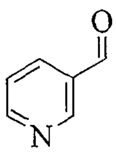 | 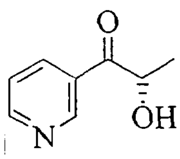 GCMS: $R_t$ = 8.03 min m/z = 151 ($M^+$) | 65 | 87 $R_t$ (*S*) = 20.14 $R_t$ (*R*) = 23.02 |
| 27. | 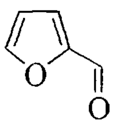 | 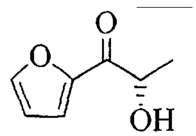 GCMS : $R_t$ = 6.42 min m/z = 140 ($M^+$) | 62 | 45 $R_t$ (*S*) = 19.69 $R_t$ (*R*) = 27.09 |
| 28. | 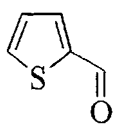 | 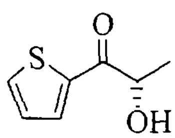 GCMS: $R_t$ =7.96 min m/z = 156 ($M^+$) | 65 | 82 $R_t$ (*S*) = 23.12 $R_t$ (*R*) = 30.73 |
| aliphatische u. olefinische Aldehyde | | | | |
| 29. | 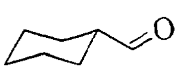 | 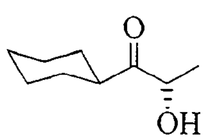 GCMS: $R_t$ =7.39 min m/z = 156 ($M^+$) | 21 | 61 $R_t$ (*S*) = 17.46 $R_t$ (*R*) = 17.85 GC Cyclodex β-I/P |
| 30. | 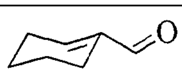 | 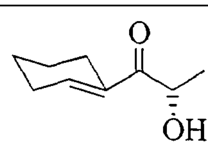 GCMS: $R_t$ = 8.01min m/z = 154 ($M^+$), | 50 | 94 $R_t$ (*S*) = 11.04 $R_t$ (*R*) = 19.93 |

Wir stellten überraschend fest, daß der Enantiomerenschuß durch Wahl des Acyldonor-Aldehyds signifikant beeinflußt werden kann. In *meta*-Position können selbst sterisch anspruchsvolle Reste, bis hin zu *iso*-Propoxy- und Benzoxy-Substituenten, eingesetzt werden. Dabei zeigte sich eine deutliche Abhängigkeit des stereochemischen Verlaufs der Reaktion von der Größe des Restes: mit steigender Raumerfüllung geht eine Zunahme der Stereoselektivität, bis hin zur Nachweisgrenze (> 99% ee), einher.
Diese erfindungsgemäße Beobachtung war v.a. deswegen unerwartet, da vergleichbare Einflüsse von Substituenten auf die optische Reinheit in PDC-katalysierten Reaktionen keinen Einfluß zeigten (Deutsche Patentanmeldung AZ. 197 36 104.8).

Einen weiteren Ansatz zur Steigerung der Enantioselektivität fanden wir in unseren reaktionstechnischen Untersuchungen zur enzymatischen Bildung von 2-HPP. Die kinetischen Untersuchungen zeigten überraschenderweise eine gegenläufige Abhängigkeit des Enantiomerenüberschusses von der Acetaldehyd und Benzaldehydkonzentration. Mit abnehmender Benzaldehyd- (Fig. 2) und zunehmender Acetaldehydkonzentration (Fig. 3) wird der Enantiomerenüberschuß erhöht. Außerdem läßt sich auch deutlich eine Abhängigkeit der Enantioselektivität von der Temperatur beobachten (Abb. 1); bei niedrigeren Temperaturen wird ein höherer Enantiomerenüberschuß erzielt. Der höchste ee von 95 % wurde bei 0 °C, 0,5 mM Benzaldehyd und 500 mM Acetaldehyd erreicht.

Hieraus lassen sich erfindungsgemäß folgende Kriterien zur Erzeugung eines hohen Enantiomerenüberschusses in der Synthese von chiralen α-Hydroxyketonen mit der Benzoylformiatdecarboxylase aus *Pseudomoas putida* ableiten:

1) möglichst niedrige Temperatur
2) hohe Acetaldehydkonzentration
3) niedrige Benzaldehydkonzentration

Die Kinetik der C-C Knüpfung wurde für das hier betrachtete System durch Anpassung der kinetischen Gleichungen an die Meßwerte mittels nicht-linearer Regression ermittelt. Die $K_M$-Werte liegen mit 310 mM (± 80 mM) für Acetaldehyd und 77 mM (± 19 mM) für Benzaldehyd sehr hoch. Bei Acetaldehydkonzentrationen größer 800 mM läßt sich eine deutliche Desaktivierung der Benzoylformiatdecarboxylase beobachten. Es konnte keine Produktinhibierung gefunden werden. Durch die rechnerische Simulation eines Satzreaktor-Versuches konnten die kinetischen Parameter verifiziert werden.

Unter den Randbedingungen einer minimalen Benzaldehyd- und einer hohen Acetaldehydkonzentration wurde zur technischen Synthese von (S) -2-Hydroxy-1-phenylpropanon ein kontinuierlich betriebener Rührkessel mit Ultrafiltrationsmembran zur Retention des Katalysators gewählt. Unter Auslaufbedingungen läßt sich hier im stationären Zustand ein festes Verhältnis von Benzaldehyd zu Acetaldehyd bei gleichzeitiger niedriger Benzaldehyd- und hoher Acetaldehydkonzentration einstellen. Die ersten Versuche wurden mit einem Enzym-Membran-Reaktor (EMR) nach Wandrey und Kula *et al*. (Bioeng. Biotechnol. 1981, 23, 2789-2802) aus Edelstahl durchgeführt. Es zeigte sich, daß die Benzoylformiatdecarboxylase in Gegenwart von Edelstahl sehr schnell desaktiviert. Aufgrund der schnellen Desaktivierung des Enzyms wurden große Umsatzbereiche überstrichen, d.h. bei sehr unterschiedlichen Benzaldehydkonzentrationen gearbeitet. Diese wurden genutzt, um die zuvor gemachten Beobachtungen bezüglich der Abhängigkeit des Enantiomerenüberschusses von den Eduktkonzentrationen unter kontinuierlichen Betriebsbedingungen zu verifizieren (Fig. 4). Bei einer deutlich geringeren Desaktivierungsrate konnte in einem Enzym-Membran-Reaktor aus Polypropylen kontinuierlich (*S*)-2-Hydroxy-1-phenyl-propanon produziert werden (Fig. 5). Da Benzaldehyd von Polypropylen absorbiert wird, wurde der Reaktor vor Inbetriebnahme solange mit Substratlösung gespült, bis Ein- und Auslauf dieselbe Konzentration aufwiesen. Der Enzym-Membran-Reaktor wurde kontinuierlich über einen Zeitraum von 180 h betrieben. Hierbei konnte (S)-2-Hydroxy-1-phenylpropanon mit einem ee von 93 % und einer Raum-Zeit-Ausbeute von 34.2 g $L^{-1}$ $d^{-1}$ (220 mmol $L^{-1}$ $d^{-1}$) produziert werden. Durch Variation der Betriebstemperatur des Rührkessels konnte ebenfalls der Einfluß der Temperatur auf den Enantiomerenüberschuß unter kontinuierlichen Betriebsbedingungen gezeigt werden (Fig. 6).

Diese Ergebnisse sind nicht durch Analogieschlüsse mit ähnlich zugänglichen enzymatischen Systemen zu erwarten, sollten sich möglicherweise aber auch auf andere übertragen lassen.

Die auf dem beschriebenem Wege zugänglichen homochiralen 2-Hydroxyketone, die zum größten Teil noch nicht in der Literatur beschrieben sind, sind für die stereoselektive organische Synthese als universell einsetzbare chirale Synthesebausteine von großer Bedeutung. So lassen sich aus ihnen durch geeignete diastereoselektive Reaktionsführung eine Bandbreite an chiralen Verbindungen auf dem Wege zu chiralen Auxiliaren bzw. Natur- und Wirkstoffen synthetisieren, die auf anderem Wege, wenn überhaupt, nur durch aufwendige und verlustreiche Schutzgruppentechniken zugänglich sind.

Zusammengefaßt läßt sich die Enantioselektivität der beschriebenen enzymatischen C-C-Verknüpfung durch verschiedene Faktoren, wie Variation der Substrate als auch durch geeignete Modifikation der Reaktionsführung, steigern.

Im Folgenden wird das erfindungsgemäße Verfahren in bevorzugten Ausführungsformen beispielhaft dargestellt. Jedoch ist das erfindungsgemäße Verfahren nicht auf die Beispiele beschränkt.
Der Einfachheit halber wird die Enzymaktivität der BFD auf die Decarboxylierung des Standards Benzoylformiat bezogen, d.h. 1 U des Enzyms ist definiert als die Menge, die 1 μmol Benzoylformiat pro Minute decarboxyliert.

Beispiel 1: Zellfreie Synthese von (S)-2-Hydroxypropiophenon ausgehend von 2-Ketocarbonsäuren bzw. Aldehyden.

Allgemeine Arbeitsweise:

Die BFD wird bevorzugt nach Reinigung des Rohextrakts mittels Anionenaustauschchromatogaphie in das erfindungsgemäße Verfahren eingesetzt. Beste Ergebnisse werden dabei durch Chromatographie an Q-Sepharose FF der Firma Pharmacia mit einem linearen NaCl-Gradient erzielt. Die BFD-Fraktion wird entsalzt und lyophilisiert.
Die enzymatischen Synthesen werden in wässerigen Puffern, üblicherweise Kaliumphosphatpuffer, durchgeführt. Durch Zugabe organischer Lösungsmittel zwischen 5-50 Volumen-% kann die Löslichkeit der aromatischen Aldehyde verbessert werden.
Der pH-Wert des Reaktionspuffers richtet sich danach, ob als Acyldonoren 2-Ketocarbonsäuren oder Aldehyde eingesetzt werden. Bei der Verwendung einer 2-Ketocarbonsäure liegt der optimale pH-wert zwischen 6,0 und 7,0, während bei der Verwendung von Aldehyden ein pH-Wert von 6,5 bis 7,5 eingestellt wird.
Neben den beschriebenen Edukten und dem Enzym BFD fügt man dem Syntheseansatz noch den Cofaktor Thiamindiphosphat, bevorzugt in einer Konzentration von 0,1 bis 5,0 mmol/l, zu.
Ferner gibt man noch Mg-Ionen, bevorzugt in Form von Magnesiumsulfat, zu, bevorzugt in einer Konzentration von 1 bis 10 mmol/l.
a) Zellfreie Synthese von (S)-2-Hydroxypropiophenon aus Benzoylformiat und Acetaldehyd in Gegenwart von BFD.

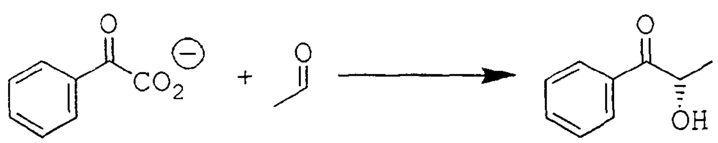

Bedingungen:

| | |
|---|---|
| Benzoylformiat: | 100 mM |
| Acetaldehyd: | 500 mM |
| Thiamindiphosphat: | 1.5 mM |
| $MgSO_4$: | 2,5 mM |
| Kaliumphosphat: | 200 mM, pH 6,5 |
| BFD: | 5 Units/ ml (bez. auf die Decarboxylierung von Benzoylformiat) |

Der pH-Wert der Reaktionsmischung wird durch dosierte Zugabe von 0,1 M Phosphorsäure konstant gehalten. Nach 18 Stunden bei 25 °C wird der Reaktionsansatz mit Chloroform extrahiert, die organische Phase über Magnesiumsulfat getrocknet, im Vakuum eingeengt und das (S)-2-HPP durch Kieselgelchromatographie (Kieselgel 60 (Merck), mobile Phase: Ether/Hexan 3:1) von überschüssigem Benzaldehyd getrennt.
(S)-2-HPP wurde mittels HPLC quantifiziert. Unter diesen Bedingungen wird aus einem 50 ml Satzreaktor 73,4 mg (S)-2-HPP isoliert.
Der Enantiomerenüberschuß beträgt 92 %.
b) Zellfreie Synthese von (S)-2-Hydroxypropiophenon aus Benzaldehyd und Acetaldehyd in Gegenwart von BFD.

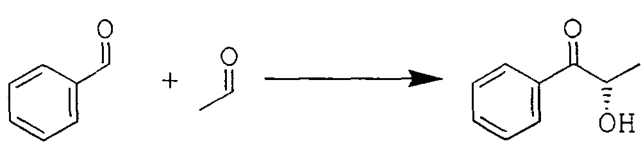

Bedingungen:

| | |
|---|---|
| Benzaldehyd: | 50 mM |
| Acetaldehyd: | 500 mM |
| Thiamindiphosphat: | 1,5 mM |
| $MgSO_4$: | 2,5 mM |
| Kaliumphosphat: | 100 mM, pH 7,0 |
| BFD: | 5 Units/ml (bez. auf die Decarboxylierung von Benzoylformiat) |

Die Reaktion wird nach 5 Stunden bei 25 °C wie oben beschrieben gestoppt.
Aus einem 50 ml Ansatz werden 150 mg (S)-2-HPP isoliert. Der Enantiomerenüberschuß beträgt 90 %.
c) Zellfreie Synthese von (R)-Benzoin aus Benzaldehyd mittels BFD

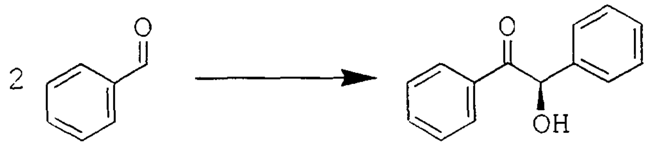

Bedingungen:

| | |
|---|---|
| Benzaldehyd: | 50 mM |
| Thiamindiphosphat: | 1,5 mM |
| $MgSO_4$: | 2,5 mM |
| Kaliumphosphat: | 100 mM, pH 7,0 |
| BFD: | 15 Units/ml (bez. auf die Decarboxylierung von Benzoylformiat) |

Nach 7 Stunden bei 25 °C wird das Benzoin aus einem 100 ml Ansatz wie oben beschrieben isoliert. Die Ausbeute beträgt 4,3 mg.

M.p. 133 °C. $[\alpha]^{D}_{25} = 109.8°$ (Aceton, c = 6,85)
ee > 98 %.

Beispiel 2: Steigerung der Enantioselektivität der BFD-katalysierten C-C-Knüpfungsreaktion durch Variation der Substrate.

Allgemeine Arbeitsweise:

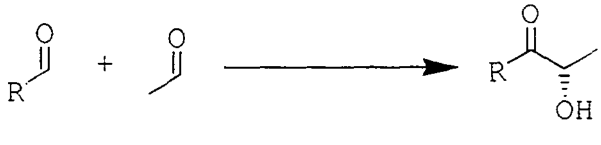

Die folgenden enzymatischen Reaktionen werden mit der Benzoylformiatdecarboxylase (BFD, EC 4.1.1.7) aus Pseudomonas putida in Form des Wild-Typ-Enzyms (BFD-wt) durchgeführt. Es wird das aufgereinigte Protein benutzt (s.o.).

Die enzymatischen Synthesen werden in einem Satzreaktor durchgeführt. Als Reaktionsgefäß dienen ein Rundkolben oder bei der Durchführung analytischer Ansätze 2 ml-Eppendorf-Reaktionsgefäße. Als Reaktionsmedium dient ein Kaliumphosphat-Puffer ($KH_2PO_4$/$K_2HPO_4$, 50 mmol/l, pH 7,0), der zusätzlich 2,5 mmol/l $MgSO_4$ und 0,5 mmol/l

Thiamindiphosphat enthält. Der Donoraldehyd (laut Tabelle 1) wird in einer Konzentration von 10 mmol/l, Acetaldehyd in einer Konzentration von 0,5 mol/l eingesetzt. Als letzte Komponente wird das Enzym BFD-wt zugegeben. Zur Stabilisierung des Enzyms wird bei präparativen Umsetzungen 0,5 mg/ml Reaktionslösung Bovine Serum Albumin (BSA) zugesetzt.
Der Reaktionsverlauf und Enantiomerenüberschuß des Produktes werden mittels HPLC an chiraler Phase (Chiralcel OB (Daicel Ltd.), Eluent n-Heptan : i-Propanol = 85:15, Fluß 0,75 ml/min, 25 °C) bzw. GC an chiraler Phase (Cyclodex β-I/P (Hewlett Packard), T(Injektor) = 200 °C, isotherm 150 °C) verfolgt. Die Produkte werden mittels NMR (AMX 300, Fa. Bruker) bzw. GCMS nachgewiesen (HP 6890 Series GC-System sowie HP 5973 Mass Selective Detector der Fa. Hewlett Packard; Säule HP-5MS (30 m x 250 μm), $T_{GC}$(Injektor) = 250 °C, $T_{MS}$ (Ionenquelle) = 200 °C, Zeitprogramm (Ofen) : $T_{0\,min}$ = 60 °C, $T_{3\,min}$ = 60 °C, $T_{14\,min}$ = 280 °C (Heizrate 20 °C/min), $T_{19\,min}$ = 280 °C). Hierzu werden 150 μl einer Probe entnommen und diese mit 150 μl n-Heptan oder Trichlomethan extrahiert. Die Phasentrennung wird durch Zentrifugieren bei 13000 rpm herbeigeführt. Die organische Phase wird direkt injiziert (HPLC, GC, GCMS).

a) Synthese von (S)-2-Hydroxy-1-(3'-methoxyphenyl)-

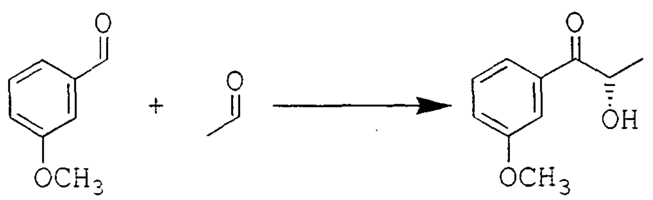

propanon

Im Folgenden wird die einfachste Form der Reaktionsführung beschrieben (Satzreaktor). Als Reaktionsgefäß dient ein Rundkolben geeigneter Größe. Während der enzymatischen Reaktion wird der Kolbeninhalt nicht oder nur langsam gerührt.

In 300 ml eines Kaliumphosphatpuffers ($KH_2PO_4/K_2HPO_4$, 50 mmol/l, pH 7,0, enthält zusätzlich 2,5 mmol/l $MgSO_4$), werden 408 mg 3-Methoxybenzaldehyd bei Raumtemperatur gelöst. Weiterhin werden in dieser Lösung 74 mg Thiamindiphosphat (z.B. Fluka Best.-Nr. 09961), 150 mg BSA und 8,5 ml Acetaldehyd gelöst. Anschließend wird die Reaktion durch Zugabe von 45 U BFD-wt oder BFD-His gestartet. Der Reaktionsverlauf wird mittels HPLC (s.o.) verfolgt. Bei einer Stagnation des Umsatzes werden erneut 45 U Enzym zugegeben. Die Reaktion wird bei Erreichen eines Umsatzes von mindestens 96 % abgebrochen. Um eine enzymbedingte Imulsionsbildung bei der Aufarbeitung mit organischen Lösungsmitteln zu vermeiden, wird die Reaktionslösung über eine Ultrafiltrationsmembran (YM 10, Amicon-Ultrafiltrationsrührzelle, 5 bar) von dem Enzym abfiltriert. Bei Verwendung des stabileren BFD-His-Enzyms kann dieses in Form von repetitiven Batchansätzen wiederverwendet werden. Die nun proteinfreie Lösung wird mit 3 x 50 ml Dichlormethan extrahiert und die organische Phase über $Na_2SO_4$ getrocknet. Das Lösungsmittel wird am Rotationsverdampfer bei 40 °C und leichtem Vakuum entfernt, worauf sich eine Trocknung im Feinvakuum ($4 \times 10^{-3}$ mbar) anschließt. Das Produkt kann racemisierungsfrei bei 4 °C aufbewahrt werden.
Ausbeute: 540 mg (quant., farbloses Öl).
$^1$H-NMR (300 MHz, $CDCl_3$, 20 °C) δ = 1.45 (d, 3H, J = 6.8 Hz, $CH_3$), 3.78 (d, 1H, J = 6.8 Hz, OH), 3.89 (s, 3H, $OCH_3$), 5.14 ('quin', 1H, J = 6.8 Hz, C<u>H</u>OH), 7.15 (d't', 1H, J = 8.0, 1.8 Hz, ar-H), 7.39 ('t', 1H, J = 8.0 Hz, ar-H), 7.45 ('t', 1H, J = 1.8 Hz, ar-H), 7.46 (m, 1H, ar-H).
$^{13}$C-NMR (75.5 MHz, $CDCl_3$, 20 °C) □ = 22.77 ($CH_3$), 55.89 ($OCH_3$), 69.82 (CHOH), 113.42, 120.73, 121.52, 130.25 (CH), 135.03, 160.35 ($C_q$), 202.70 (CO).
MS(EI): m/z = 180.1 ($M^+$, 22 %), 135.1 ($M^+$-$C_9H_5O$, 100 %), 107.1 ($M^+$-CO- $C_2H_5O$, 32 %), 77.0 ($C_6H_5^+$, 25 %).
HRMS: $C_{10}H_{12}O_3$; kalk. 180.0787, gef. 180.0782
CHN-Bestimmung: $C_{10}H_{12}O_3$; kalk. C 66.65 %, H 6.71 %, gef. C 66.27 %, H 6.64 %.
ee(HPLC) = 96 %, $R_t$(S) = 18.24 min, $R_t$(R) = 34.06 min.
$[\alpha]_D^{25}$ =-65.24° ($CHCl_3$, c = 1.13) ($[\alpha_0]_D^{25}$ = -68.53°).

b) Synthese von (S)-2-Hydroxy-1-(3'-isopropyloxyphenyl)-propanon

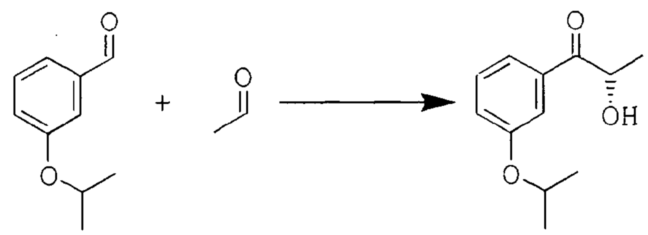

Die Reaktionsführung wird analog Beispiel 1 gewählt. Anstelle von 3-Methoxybenzaldehyd werden 164 mg 3-Isopropyloxybenzaldehyd als Substrat eingesetzt. 3-Isopropyloxybenzaldehyd ist nach einer literaturbekannten Arbeitsvorschrift erhältlich (Morris, J. Org. Chem., 1950, 1913). Die anderen Komponenten der Reaktion werden entsprechend der kleineren Ansatzgröße wie folgt variiert: Kaliumphosphatpuffer (200 ml), Thiamindiphosphat (50 mg), BSA (100 mg), Acetaldehyd (5.7 ml).
Ausbeute: 190 mg (91 %, farbloses Öl).
$^{1}$H-NMR (300 MHz, $CDCl_3$, 20 °C) $\delta$ = 1.36 (d, 6H, J = 6.1 Hz, $CH(C\underline{H}_3)_2$), 1.43 (d, 3H, J = 6.8 Hz, $CH_3$), 3.77 (d, 1H, J = 6.8 Hz, OH), 4.62 (sept, 1H, J = 6.1 Hz, $C\underline{H}(CH_3)_2$), 5.12 ('quin', 1H, J = 6.8 Hz, $C\underline{H}OH$), 7.12 (d, 1H, J = 8.0, ar-H), 7.38 ('t', 1H, J = 8.0 Hz, ar-H), 7.45 (m, 2H, ar-H).
$^{13}$C-NMR (75.5 MHz, $CDCl_3$, 20 °C) □ = 22.11, 22.13 ($CH(\underline{C}H_3)_2$), 22.62 ($CH_3$), 69.61 ($\underline{C}H(CH_3)_2$), 70.46 (CHOH), 115.31, 121.03, 122.05, 130.10 (CH), 134.82, 158.49 ($C_q$), 202.52 (CO).
MS(EI): m/z = 208.1 ($M^+$, 25 %), 163.1 ($M^+$-$C_2H_5O$, 100 %), 121.1 ($M^+$-$C_3H_6$-$C_2H_5O$, 98 %), 95.0 ($C_5H_3O_2^+$, 45 %), 77.0 ($C_6H_5^+$, 16 %), 65.0 ($C_5H_5^+$, 19 %).
HRMS: $C_{12}H_{16}O_3$; kalk. 208.1099, gef. 208.1102
CHN-Bestimmung: $C_{12}H_{16}O_3$; kalk. C 69.21 %, H 7.74 %, gef. C 69.10 %, H 7.74 %.
ee(HPLC) = 99.3 %, $R_t$(S) = 16.78 min, $R_t$(R) = 24.16 min (Eluent i-Hexan : i-Propanol =9:1).
$[\alpha]_D^{25}$ = -54.88° ($CHCl_3$, c = 1.45).

Beispiel 3: Synthese von (S)-2-Hydroxy-1-phenyl-propanon unter Berücksichtigung des Einflusses der Substratkonzentrationen und der Temperatur.

a) Synthese von (S)-2-Hydroxy-1-phenyl-propanon ((S)-2-HPP) mit ee = 90 %

Die Reaktion wird in einem Satzreaktor mit einem Reaktionsvolumen von 50 ml bei 25 °C durchgeführt. Es werden 50 mM Benzaldehyd und 500 mM Acetaldehyd in Phosphatpuffer(50 mM, pH 7,0, enthält zusätzlich 0,5 mM Thiamindiphosphat und 0,05 mM $MgCl_2$) eingesetzt. Die Reaktion wird durch Zugabe von 50 U Benzoylformiatdecarboxylase gestartet. Die Reaktion wurde nach 5 h gestoppt und (S)-2-HPP mit Hexan oder Trichlormethan extrahiert. Die organische Phase wird durch Zentrifugation bei 13.000 rpm abgetrennt und über $MgSO_4$ getrocknet.
Das Lösungsmittel wird im Vakuum entfernt und das Rohprodukt durch Säulenchromatographie ($SiO_2$, Diethylether/n-Hexan (3:1, v/v), $R_f$ = 0.49) aufgereinigt. Es werden 150 mg (1 mmol) (S)-2-HPP (40 % Ausbeute) mit einem ee = 90 % erhalten.

b) Synthese von (S)-2-Hydroxy-1-phenylpropanon ((S)-2-HPP) mit ee=95 %

Die Reaktion wird in einem Satzreaktor bei 0 °C durchgeführt. Es werden 0,5 mM Benzaldehyd, 500 mM Acetaldehyd 0,5 mM ThDP und 0,05 mM $MgCl_2$ in 50 mM Phosphatpuffer, pH 7, eingesetzt. Die Reaktion wird durch Zugabe von 0,17 U/ml Benzoylformiatdecarboxylase gestartet. Es wird (S)-2-HPP mit einem ee = 95 % erhalten.

Beispiel 4: Kontinuierliche Synthese im Enzym-Membran-Reaktor.

Allgemeine Anleitung:

Es wird ein Enzym-Membran-Reaktor (EMR) aus VA-Stahl bzw. aus Polypropylen mit einem Volumen von 10 ml eingesetzt. Alle Schlauchverbindungen sind aus Teflon. Dem Reaktor sind eine Blasenfalle und ein Manometer vorgeschaltet. Der Auslauf wird in Fraktionen gesammelt. Ein zunächst vorgeschalteter Sterilfilter erweist sich als lösungsmittelunbeständig und wird daher nicht weiter verwendet. Die Substratlösungen sind aufgrund der sehr hohen Aldehydkonzentrationen autosteril.

Die Membranen werden vor der Verwendung in Wasser eingelegt und im Reaktor mit 5 mg/ml BSA vorbelegt. Das System wird solange mit der Substratlösung gespült, bis Ein- und Auslaufkonzentration identisch sind, um eventuelle

Absorptionseffekte der eingesetzten Materialien auszuschließen. Die Reaktion wird dann durch Injektion der Enzymlösung in das Septum der Blasenfalle gestartet.

a) Kontinuierliche (S)-2-Hydroxy-1-phenylpropanon Synthese im Stahlreaktor

Als Substratlösung wird 40 mM Benzaldehyd, 500 mM Acetaldehyd, 0,5 mM ThDP und 1 mM $MgCl_2$ in 50 mM Phosphatpuffer bei pH 7 angesetzt. Der Reaktor wird durch Injektion von 65 U BFD gestartet. Betrieben wird der EMR bei einer Verweilzeit von 1 bzw. 2 h.

b) Kontinuierliche (S)-2-Hydroxy-1-phenyl-propanon Synthese im Polypropylenreaktor

Als Substratlösung wird 10 mM Benzaldehyd, 100 mM Acetaldehyd, 4 mM ThDP und 0,5 mM $MgCl_2$ in 50 mM Phosphatpuffer bei pH 7 angesetzt.
Der Reaktor wird durch Injektion von 50 U BFD gestartet. Betrieben wird der EMR unter folgenden Reaktionsbedingungen:

| Prozeßzeit/h | Verweilzeit/h | Temperatur/˚C |
|---|---|---|
| 0-23 | 1 | 25 |
| 23-47 | 1 | 10 |
| 47-76 | 2 | 10 |
| 76-180 | 2 | 4 |

## Patentansprüche

1. Verfahren zur stereoselektiven Synthese von 2-Hydroxyketonen ,
**dadurch gekennzeichnet, dass**

    - als ein erstes Substrat ein Aldehyd, ausgenommen Benzaldehyd, oder eine 2-Ketosäure, ausgenommen Benzoylformiat, eingesetzt wird,
    - als ein zweites Substrat ein Aldehyd, ausgenommen Benzaldehyd, eingesetzt wird,
    - die Synthese in Gegenwart von Benzoylformiatdecarboxylase stattfindet.
    - zur Steigerung der Enantioselektivität die Temperatur während des Verfahrens weniger als 30 ˚C, insbesondere weniger als 25 ˚C, und mehr als -10˚C beträgt.
    - zur Steigerung der Enantioselektivität die Konzentration des ersten Substrates weniger als 500 mM, insbesondere weniger als 50 mM, jedoch mehr als 1 μM beträgt und die Konzentration des zweiten Substrates weniger als 1 M, insbesondere weniger als 500 mM, und mehr als 1 mM beträgt.

2. Verfahren zur stereoselektiven Synthese von 2-Hydroxyketone nach vorhergehendem Anspruch,
**dadurch gekennzeichnet, dass**
als Enzym eine Benzoylformiatdecarboxylase (BFD) aus *Pseudomonas putida* eingesetzt wird.

3. Verfahren zur stereoselektiven Synthese von 2-Hydroxyketone nach Anspruch 1,
**dadurch gekennzeichnet, dass**
als ein zweites Substrat ein Acetaldehyd eingesetzt wird.

4. Verfahren zur stereoselektiven Synthese von 2-Hydroxyketone nach Anspruch 1,
**dadurch gekennzeichnet, dass**
als ein erstes Substrat ein aromatisches Aldehyd eingesetzt wird.

5. Verfahren zur stereoselektiven Synthese von 2-Hydroxyketone nach vorhergehendem Anspruch,
**dadurch gekennzeichnet, dass**
als ein erstes Substrat ein substituiertes aromatisches Aldehyd eingesetzt wird.

6. Verfahren zur stereoselektiven Synthese von 2-Hydroxyketone nach vorhergehendem Anspruch,
**dadurch gekennzeichnet, dass**
als ein erstes Substrat ein substituiertes heteroaromatisches Aldehyd eingesetzt wird.

**7.** Verfahren zur stereoselektiven Synthese von 2-Hydroxyketone nach Anspruch 1,
**dadurch gekennzeichnet, dass**
als ein erstes Substrat ein nichtaromatisches Aldehyd eingesetzt wird.

**8.** Verfahren zur stereoselektiven Synthese von 2-Hydroxyketone nach Anspruch 9,
**dadurch gekennzeichnet, dass**
als ein erstes Substrat ein lineares, nichtaromatisches Aldehyd eingesetzt wird.

**9.** Verfahren zur stereoselektiven Synthese von 2-Hydroxyketone nach Anspruch 9,
**dadurch gekennzeichnet, dass**
als ein erstes Substrat ein cyclisches, nichtaromatisches Aldehyd eingesetzt wird.

**10.** Verfahren zur stereoselektiven Synthese von 2-Hydroxyketone nach vorhergehendem Anspruch,
**dadurch gekennzeichnet, dass**
als ein erstes Substrat ein cyclisches, nichtaromatisches hetero-Aldehyd eingesetzt wird.

## Claims

**1.** Method for the stereoselective synthesis of 2-hydroxyketones, **characterised in that**

- an aldehyde, except for benzaldehyde, or a 2-keto acid, except for benzoyl formate, is used as a first substrate,
- an aldehyde, except for benzaldehyde, is used as a second substrate;
- the synthesis takes place in the presence of benzoylformate decarboxylase;
- to increase the enantioselectivity, the temperature is less than 30˚C, and in particular is less than 25˚C, and is more than -10˚C, during the method;
- to increase the enantioselectivity, the concentration of the first substrate is less than 500 mM, and in particular is less than 50 mM, but is more than 1 μM, and the concentration of the second substrate is less than 1 M, and in particular is less than 500 mM, and is more than 1 mM.

**2.** Method for the stereoselective synthesis of 2-hydroxyketones according to the preceding claim, **characterised in that** a benzoylformate decarboxylase (BFD) from *Pseudomonas putida* is used as an enzyme.

**3.** Method for the stereoselective synthesis of 2-hydroxyketones according to claim 1, **characterised in that** an acetaldehyde is used as a second substrate.

**4.** Method for the stereoselective synthesis of 2-hydroxyketones according to claim 1, **characterised in that** an aromatic aldehyde is used as a first substrate.

**5.** Method for the stereoselective synthesis of 2-hydroxyketones according to a preceding claim, **characterised in that** a substituted aromatic aldehyde is used as a first substrate.

**6.** Method for the stereoselective synthesis of 2-hydroxyketones according to a preceding claim, **characterised in that** a substituted heteroaromatic aldehyde is used as a first substrate.

**7.** Method for the stereoselective synthesis of 2-hydroxyketones according to claim 1, **characterised in that** a non-aromatic aldehyde is used as a first substrate.

**8.** Method for the stereoselective synthesis of 2-hydroxyketones according to claim 9, **characterised in that** a linear non-aromatic aldehyde is used as a first substrate.

**9.** Method for the stereoselective synthesis of 2-hydroxyketones according to claim 9, **characterised in that** a cyclic non-aromatic aldehyde is used as a first substrate.

**10.** Method for the stereoselective synthesis of 2-hydroxyketones according to a preceding claim, **characterised in that** a cyclic non-aromatic heteroaldehyde is used as a first substrate.

## Revendications

1. Procédé de synthèse stéréosélective de 2-hydroxycétones, **caractérisé en ce que**

   - on utilise comme premier substrat un aldéhyde, à l'exception du benzaldéhyde, ou un 2-cétoacide à l'exception du formiate de benzoyle,
   - on utilise comme deuxième substrat un aldéhyde, à l'exception du benzaldéhyde,
   - la synthèse a lieu en la présence de benzoylformiatedécarboxylase,
   - pour augmenter la sélectivité énantiomérique, la température pendant le procédé est inférieure à 30˚C, notamment inférieure à 25˚C, et est supérieure à -10˚C,
   - pour augmenter la sélectivité énantiomérique, la concentration du premier substrat est inférieure à 500 mM, notamment inférieure à 50 mM, mais supérieure à 1 μM et la concentration du deuxième substrat est inférieure à 1 M, notamment inférieure à 500 mM, et supérieure à 1 mM.

2. Procédé de synthèse stéréosélective de 2-hydroxycétones suivant la revendication précédente, **caractérisé en ce que** on utilise comme enzyme une benzoylformiatedécarboxylase (BFD) provenant de *pseudomonas putida.*

3. Procédé de synthèse stéréosélective de 2-hydroxycétones suivant la revendication 1, **caractérisé en ce que** l'on utilise comme deuxième substrat un acétaldéhyde.

4. Procédé de synthèse stéréosélective de 2-hydroxycétones suivant la revendication 1, **caractérisé en ce que** l'on utilise comme premier substrat un aldéhyde aromatique.

5. Procédé de synthèse stéréosélective de 2-hydroxycétones suivant la revendication précédente, **caractérisé en ce que** l'on utilise comme premier substrat un aldéhyde aromatique substitué.

6. Procédé de synthèse stéréosélective de 2-hydroxycétones suivant la revendication précédente, **caractérisé en ce que** l'on utilise comme premier substrat un aldéhyde hétéroaromatique substitué.

7. Procédé de synthèse stéréosélective de 2-hydroxycétones suivant la revendication 1, **caractérisé en ce que** l'on utilise comme premier substrat un aldéhyde non aromatique.

8. Procédé de synthèse stéréosélective de 2-hydroxycétones suivant la revendication 9, **caractérisé en ce que** l'on utilise comme premier substrat un aldéhyde non aromatique linéaire.

9. Procédé de synthèse stéréosélective de 2-hydroxycétones suivant la revendication 4, **caractérisé en ce que** l'on utilise comme premier substrat un aldéhyde non aromatique cyclique.

10. Procédé de synthèse stéréosélective de 2-hydroxycétones suivant la revendication précédente, **caractérisé en ce que** l'on utilise comme premier substrat un hétéroaldéhyde non aromatique cyclique.

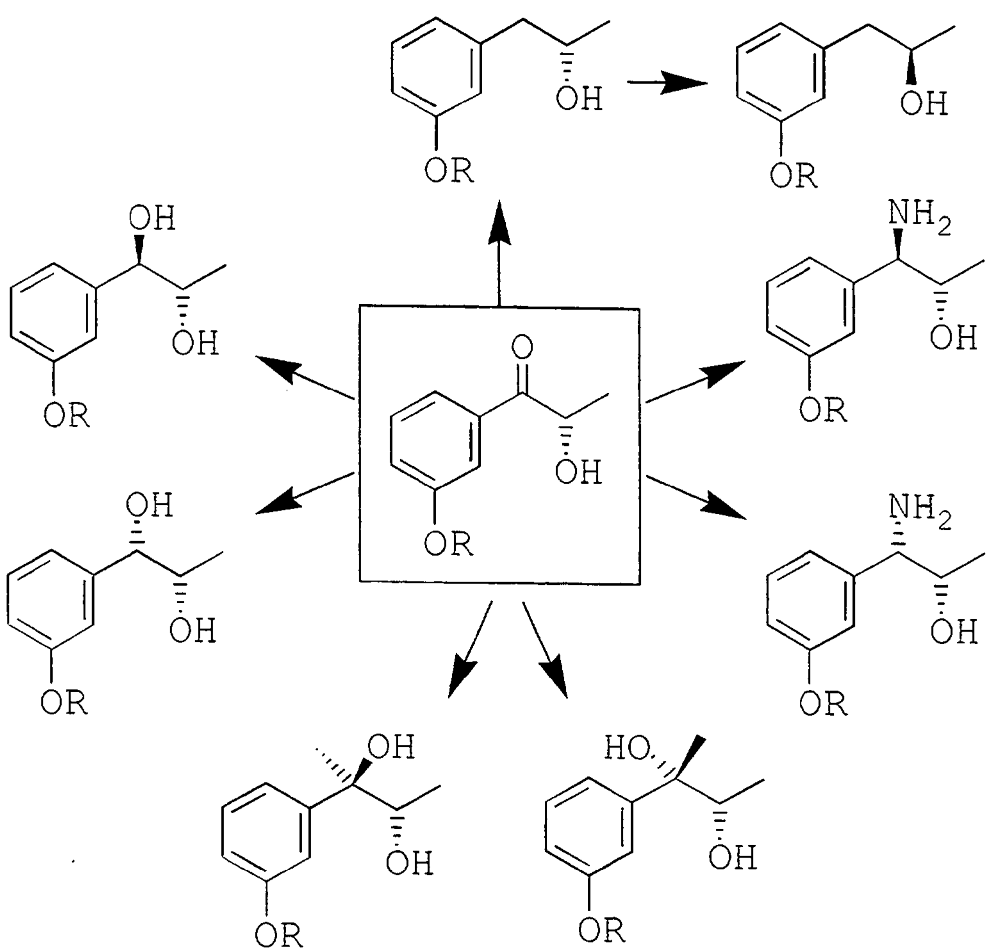

Figur 1

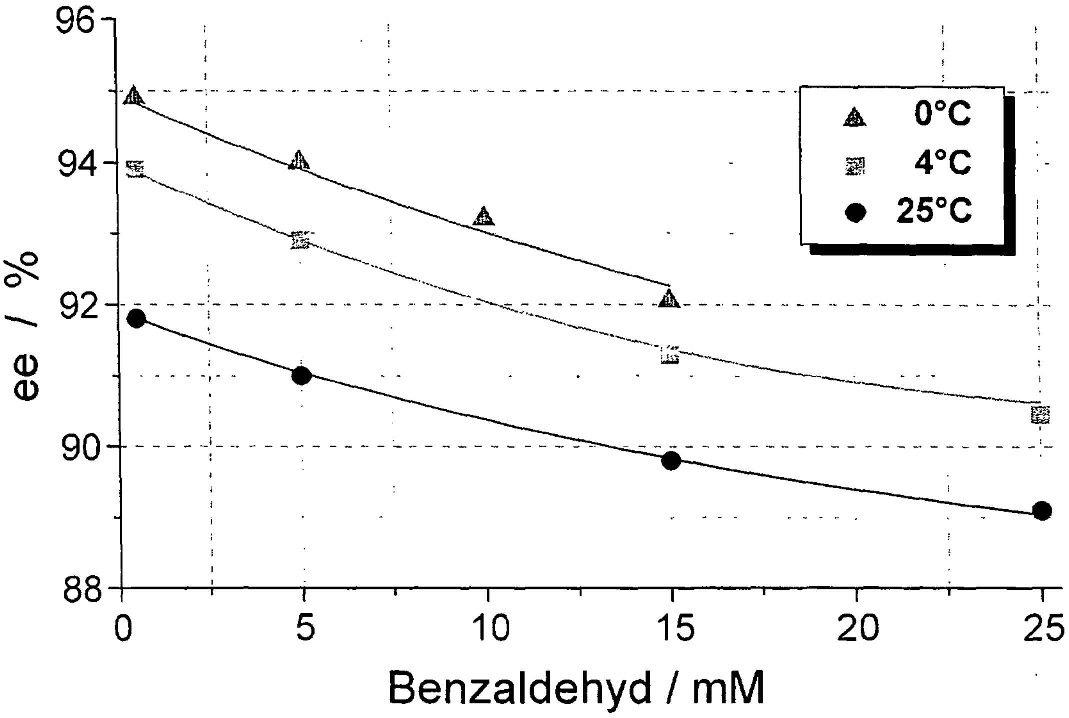


Figur 2

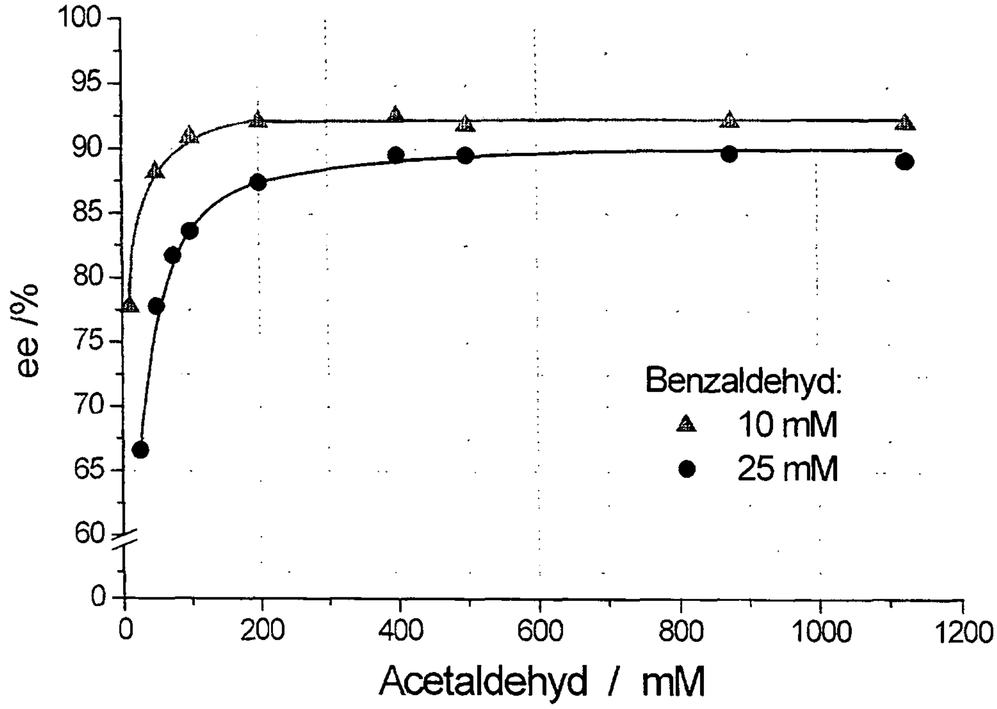
100
95
90
85
80
75
70
65
60
0
ee /%
0
200
400
600
800
1000
1200
Acetaldehyd / mM
Benzaldehyd:
10 mM
25 mM

Figur 3

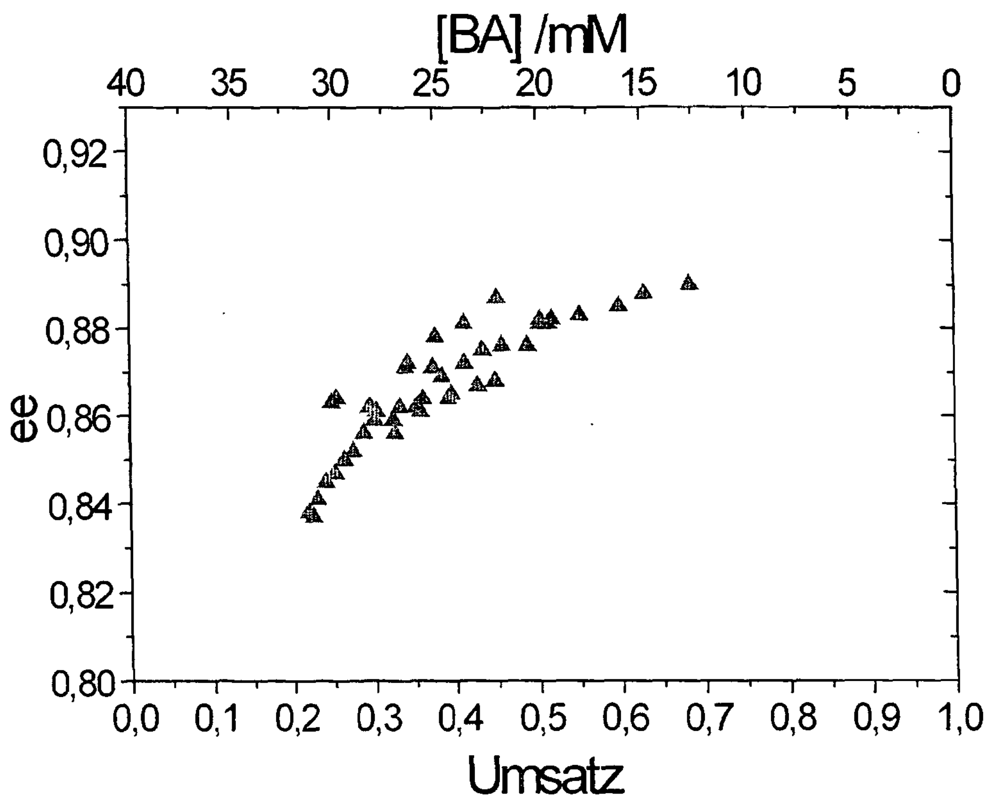
[BA] /mM
40
35
30
25
20
15
10
5
0
0,92
0,90
0,88
0,86
0,84
0,82
0,80
ee
0,0
0,1
0,2
0,3
0,4
0,5
0,6
0,7
0,8
0,9
1,0
Umsatz

Figur 4

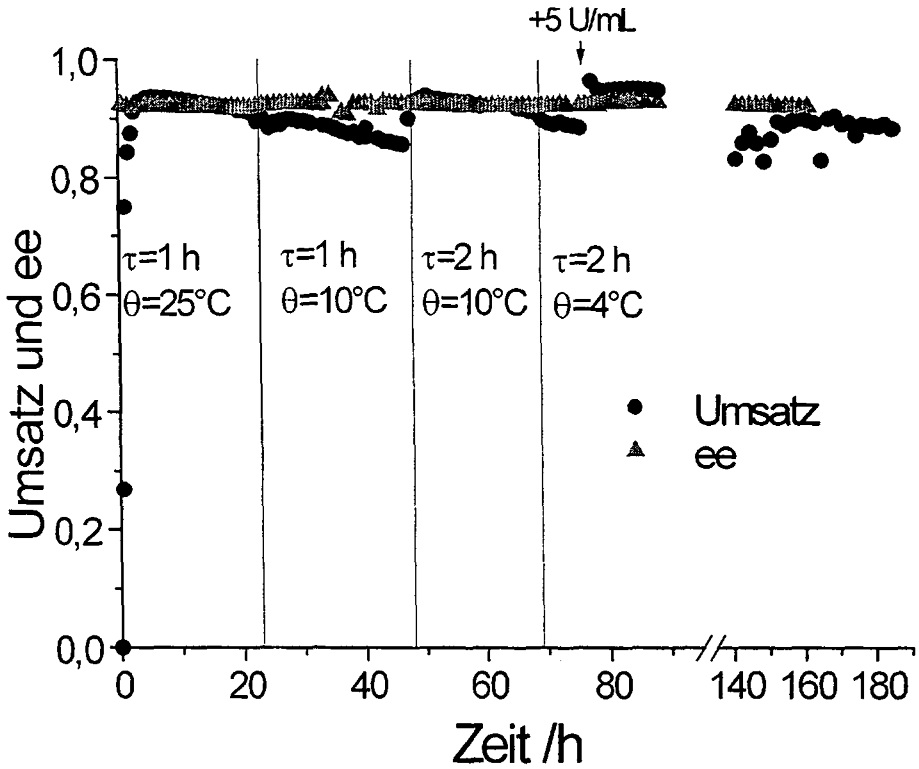
+5 U/mL
1,0
0,8
0,6
0,4
0,2
0,0
Umsatz und ee
τ=1 h
θ=25°C
τ=1 h
θ=10°C
τ=2 h
θ=10°C
τ=2 h
θ=4°C
Umsatz
ee
0
20
40
60
80
140 160 180
Zeit /h

Figur 5

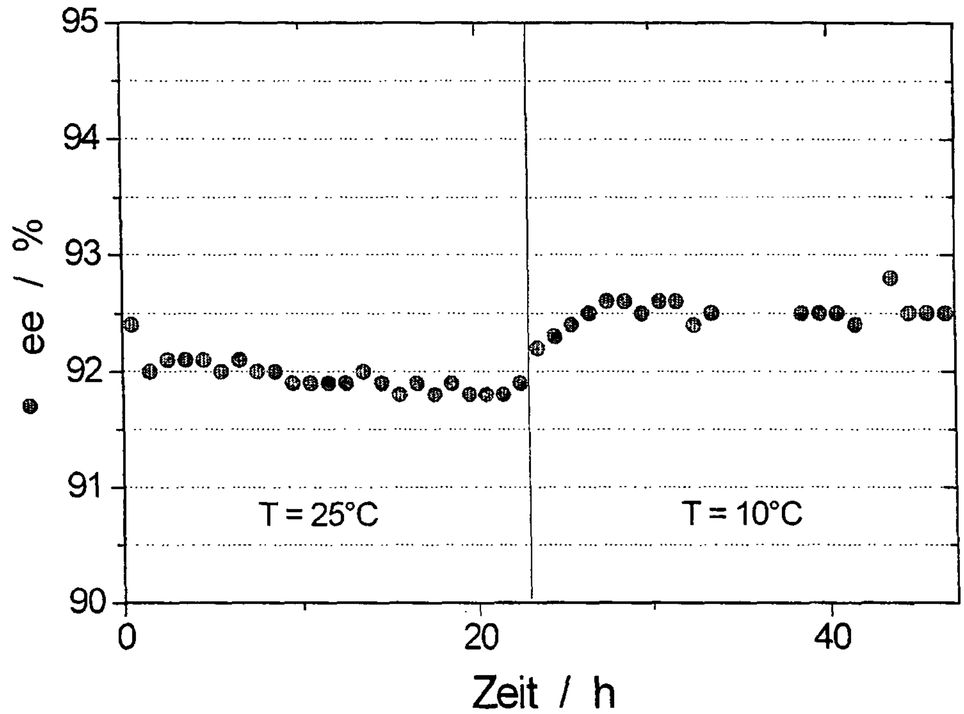


Figur 6